# EUROPEAN PATENT SPECIFICATION

(11) **EP 3 215 081 B1**
(45) Date of publication and mention of the grant of the patent: **11.12.2024**
(21) Application number: 15857643.9
(22) Date of filing: 22.10.2015
(51) Int. Cl.: A61F 9/04, A61F 6/14, A61N 5/06

(54) **SLEEP MASK THAT INCORPORATES LIGHT TO REGULATE UTERINE CONTRACTIONS**
SCHLAFBRILLE MIT LICHT ZUR REGULIERUNG VON GEBÄRMUTTERKONTRAKTIONEN
MASQUE DE SOMMEIL QUI COMPREND UN ÉCLAIRAGE POUR RÉGULER LES CONTRACTIONS UTÉRINES

(30) Priority: 04.11.2014 WO PCT/US2014/063877
(43) Date of publication of application: 13.09.2017
(73) Proprietor: The Florida State University Research Foundation, Inc., Tallahassee, Florida 32306-4391 (US)
(72) Inventor: OLCESE, James, Tallahassee, FL 32309 (US)
(74) Representative: Stanley, David William
(86) International application number: PCT/US2015/056923
(87) International publication number: WO 2016/073213

(56) References cited:
- WO-A1-2014/066059
- WO-A2-2015/073259
- WO-A2-2015/073259
- US-A1- 2003 172 878
- US-A1- 2010 171 441
- US-A1- 2012 053 395
- US-A1- 2012 209 355
- US-A1- 2013 053 929
- US-A1- 2013 060 306
- US-A1- 2014 094 877
- MARIANA FIGUEIRO ET AL: "A train of blue light pulses delivered through closed eyelids suppresses melatonin and phase shifts the human circadian system", NATURE AND SCIENCE OF SLEEP, 1 October 2013 (2013-10-01), pages 133, XP055340644, DOI: 10.2147/NSS.S52203
- MARIANA G FIGUEIRO ET AL: "Preliminary evidence that light through the eyelids can suppress melatonin and phase shift dim light melatonin onset", BMC RESEARCH NOTES, BIOMED CENTRAL LTD, GB, vol. 5, no. 5, 7 May 2012 (2012-05-07), pages 221, XP021108141, ISSN: 1756-0500, DOI: 10.1186/1756-0500-5-221

## Description

### Field of the Invention

The invention relates to the field of pregnancy and, more particularly, to regulating and suppressing uterine contractions in a pregnant human female.

### Background of the Invention

In Western societies preterm labor occurs in more than 12% of all pregnancies. It remains a major cause of perinatal morbidity and is associated with 70% of neonatal mortality. According to the Institute of Medicine of the National Academies of Sciences, the economic burden of preterm births in the United States is well over $26 billion per year (>$ 00,000 per infant). Despite this continually increasing medical challenge there has been relatively little progress in the past 20 years in understanding the processes initiating labor, whether term and preterm.

There is no test to accurately predict preterm labor. The primary goal in preventing preterm birth is to eliminate the high risk of neonatal mortality and neonatal complications (especially in terms of pulmonary and brain function). Among the major pharmacological approaches for treating preterm labor are oxytocin receptor antagonists (Atosiban^{®}; Tractocile^{®}), β-adrenergic receptor agonists, cyclooxygenase (COX) inhibitors, nitric oxide donors and magnesium sulfate. Although some of these agents have modest success as tocolytics, many have considerable, sometimes serious, side effects which can limit their use. Although progesterone has been used to prevent preterm labor in women at risk, most preterm births occur in women with no significant risk factors.

Clearly, this socio-medically important problem is far from resolved. Understanding the molecular mechanisms of labor thus should have a high priority in biomedicine. There is evidence of a synergistic action between melatonin receptor activation and oxytocin-induced signaling that may provide a key hormonal event in the initiation of labor. Ramifications of these findings for the practice of obstetrics could be dramatic. For example, the blockade of melatonin receptor activity might be of great value in preventing preterm labor and thus extending pregnancy to improve the chances of optimal survival for the newborn.

U.S. Patent No. 8,445,436 by the same inventor, discloses that the brain hormones melatonin and oxytocin use similar intracellular mechanisms in promoting contraction of human myometrium smooth muscle cells. Oxytocin analogues are important tools in obstetrical practice, e.g. infusion of oxytocin analogues is commonly used to induce labor, while oxytocin antagonists are used to prolong pregnancy in cases of preterm labor (although they are only minimally effective). A significant positive synergistic action of melatonin and oxytocin exists on human myometrial cell contractions in vitro, such that in the presence of melatonin, even as little as 1 % of the oxytocin dose normally needed for maximal contraction is fully effective. These findings could lead to the development of new melatonin plus low dose oxytocin combinations for labor induction without the mentioned side effects of high oxytocin levels.

Signaling through uterine melatonin receptors may actively contribute to labor by serving to temporally "gate" the events leading to uterine contractions at night. Endogenous melatonin may normally act synergistically with oxytocin (and potentially other pro-contractile factors) to facilitate the coordinated and forceful contractions of the pregnant uterus necessary for term labor. By extension, expression of these receptors prematurely in the myometrium of pregnant women may contribute to preterm labor. It has also been found that bright light exposure will impact melatonin.

WO 2015/073259 discloses a sleep mask that incorporates light to regulate uterine contractions. US 2012/209355 discloses a sleep mask configured to provide light therapy to a subject. Light delivered through closed eyelids by means of a light mask to suppress melatonin is discussed in 'Mariana Figueiro et al: "A train of blue light pulses delivered through closed eyelids suppresses melatonin and phase shifts the human circadian system", Nature and Science of Sleep, 1 October 2013 (2013-10-01), page 133, XP055340644, DOI: 10.2147/NSS.S52203'; and also in Mariana G Figueiro et al: "Preliminary evidence that light through the eyelids can suppress melatonin and phase shift dim light melatonin onset", BMC RESEARCH NOTES, BIOMED CENTRAL LTD, GB, vol. 5, no. 5, 7 May 2012 (2012-05-07), page 221, XP021108141, ISSN: 1756-0500, DOI: 10.1186/1756-0500-5-221.

### Summary of the Invention

According to the present invention, there is provided a sleep mask for decreasing endogenous melatonin levels to aid in suppressing uterine contractions in a pregnant female wearer, comprising: a flexible eye cover configured to be worn by a pregnant female wearer and configured to cover and shield the eyes of the wearer from ambient light; at least one light source carried by the flexible eye cover and positioned to emit light having a wavelength of 450 to 570 nm onto the eyelids of the wearer when asleep and penetrate the eyelids when closed; and a controller connected to the at least one light source and configured to activate the at Least one light source: wherein the light irradiance emitted from the at least one light source is between about 0.1 to 0.5 W/m².

The wavelength of the light emitted from the at least one light source may be between about 450 to 500 nm. The controller may be configured to increase the illumination of the light emitted from the at least one light source over a predetermined period of time. The illumination may be increased over a time period from between about 5 to 30 seconds. The controller may be configured to pulse the light emitted from the at least one light source in discrete on and off cycles. The light may be pulsed for about 1 to 3 seconds per minute for about 30 to about 60 minutes.

The light source may comprise a light emitting diode (LED) carried by the flexible eye cover and positioned at a location where an LED can emit light onto the eyelids of the wearer when asleep. The controller may be configured to control the at least one light source to direct light intermittently over a period of time into the eyes and at a wavelength to shift the biologic time clock of the human female.

At least one sensor may be carried by the flexible eye cover and positioned to engage the skin of the wearer and measure at least one of EMG (electromyogram), EEG (electroencephalography), and temperature: wherein the controller is connected to the at least one sensor and configured to control at least one of the duration, wavelength and irradiance of the light emitted from the light source based on at least one of sensed EMG, EEG and temperature. The at least one sensor may comprise a sensor array comprising the EEG sensor, the EMG sensor and the temperature sensor. The sensor array may further comprise at least one of an EOG (electrooculogram) sensor and a pulse oximetry sensor.

### Brief Description of the Drawings

FIG. 1 is a bar graph showing the number of contractions per hour a pregnant volunteer experienced overnight and how the number changed when a lamp was turned on for about one hour.
FIG. 2 is a bar graph showing the number of contractions per hour that another pregnant volunteer experienced overnight and how the number changed when a lamp was turned on for about one hour.
FIG. 3 is a line graph showing composite results of the melatonin levels of five pregnant volunteers when a lamp was turned on for about an hour.
FIG. 4 is a fragmentary perspective view of a user wearing a pair of goggleslglasses that may be worn by a pregnant female during nocturnal hours to regulate uterine contractions while she sleeps.
FIG. 5 is a rear fragmentary view of a sleep mask formed as a flexible eye cover that can be worn by a pregnant female and includes a source of light as LED's and a processor that activates the light in a manner to decrease endogenous melatonin levels to aid in regulating uterine contractions in a pregnant female.
FIG. 6 is a front fragmentary view of the sleep mask showing part of the fabric forming the flexible eye cover and broken away to show the position of a processor and battery and relative positioning of the LED's and also showing a separate wireless power source and controller that may be used in conjunction with the sleep mask.
FIG. 7 is a fragmentary perspective view of a female wearing another embodiment of the sleep mask that includes at least one sensor in accordance with a non-limiting example.
FIG. 8 is a block diagram of the components used in the sleep mask shown in FIG. 7 in accordance with a non-limiting example.
FIG. 9 is a fragmentary, plan view of a system for use in a facility that treats pregnant human females in accordance with a non-limiting example.

### Detailed Description

In the Detailed Description, reference is made to particular features of embodiments of the invention. Where a particular feature is disclosed in the context of a particular aspect or embodiment of the invention, that feature can also be used, to the extent possible, in combination with and/or in the context of other particular aspects and embodiments of the invention, and in the invention generally, within the scope of the invention as defined in the appended claims.

Embodiments of the invention may be used to regulate uterine contractions by exposing pregnant females to light. The term "regulate" in this context means reducing the number of uterine contractions over a given time period, reducing the intensity of the uterine contractions, and or preventing uterine contractions from occurring when they might otherwise occur in the absence of light. These methods are useful, for example, to prolong pregnancy, prevent preterm birth, or, if preterm birth is inevitable, to delay the preterm birth.

To show that removing melatonin's drive to the pregnant myometrium can lower uterine contractions during pregnancy, clinical studies had been completed in which >39 week term pregnant volunteers were recruited. The volunteers were continuously monitored for uterine contractions from 7:00 p.m. until 7:00 a.m. under dim light. At 11:00 p.m., a 10,000 lux full spectrum lamp 1 meter from the head was activated for 1 hour to suppress melatonin secretion. This study is disclosed in U.S. patent number 8,992,589 by the same inventor.

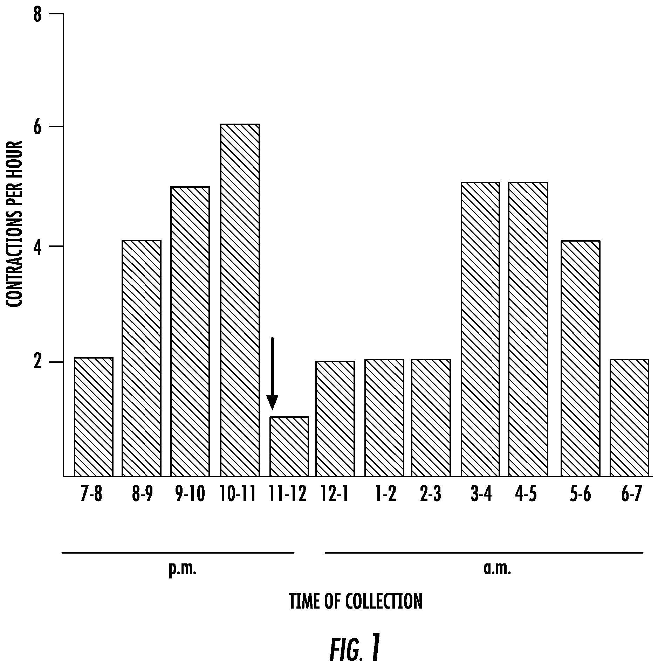

As shown in FIGS 1-3 (representative of 18 volunteers assessed), nocturnal contraction frequency rises early in the night, only to be suppressed by bright light exposure. Once endogenous melatonin levels return to high nocturnal levels (2-3 hours later), uterine contractions are again near maximal. These results support the view that melatonin plays a key role in the uterine contractions of labor. It would be advantageous if a light emitting device could be used to treat preterm labor and is amenable for use by a pregnant woman at night during sleep.

Parturition is a physiological process that occurs when pregnant females are in labor. It is characterized by increasingly frequent uterine contractions and cervical effacement, which ultimately leads to delivery of offspring. Parturition is a complex physiological and molecular biological process that has evolved differently in different species due to each species' unique environmental and temporal niches. Most mammals have adapted to selective pressures, such as the availability of food and prevalence of predators, by developing either a diurnal or nocturnal activity phase. Pregnant females have adapted to deliver their offspring in their den or home camp rather than in the field, which enhances their safety, security, and birth success.

The selective advantage for entering parturition during the daytime or nighttime phases is reflected in the differential timing of this event among many species of nocturnal rodents and diurnal mammals, such as sheep and primates, Rats, for example, enter parturition predominantly during the day time, even when the light-dark cycles are reversed.[1-3] Similarly, golden hamsters develop strong uterine contractions and deliver their young during daytime hours.[4] Humans, on the other hand, tend to enter labor during the late nighttime and early morning hours [5-9] with parturition typically following 12 to 24 hours thereafter, at least in nulliparous women.[7] The frequency of uterine contractions in preterm women at risk for spontaneous premature delivery increases significantly at night.[10]

In non-human primates, the late-term myometrial contractions and the sensitivity of the uterus to the contractile effects of oxytocin have been shown to be the highest early in the night phase.[11-13] In addition, the phasing of nocturnal parturition in nonhuman primates has been shown to also be shifted by reversal of the light/dark cycles [14], pointing to a light-sensitive clock mechanism underlying parturition. Since both humans and nonhuman primates show nocturnally peaking uterine contractions in late-term pregnancy [15-17], the intriguing question arises-what are the circadian signals that drive nocturnal uterine activity in late term human pregnancy?

Maintenance of appropriate circadian phase in peripheral tissues requires zeitgebers (entraining cues) that are coupled with the central circadian oscillator in the brain's suprachiasmatic nuclei (SCN) via neural pathways, rhythmic endocrine, and/or metabolic signals. For many peripheral clocks, such as the liver, heart, pancreas, and so on, autonomically driven neuroendocrine output cues such as melatonin and glucocorticoids are considered to play a key role. [18] Evidence continues to accumulate showing that these two hormones have significant effects on the endogenous circadian clockwork in a variety of peripheral tissues.[19-24]

It is known that the endogenous melatonin level of a typical person rises gradually from about 9:00 p.m. to a maximum at about 2:00 a.m. After about 2:00 a.m., the endogenous melatonin level gradually decreases until morning and remains very low throughout the day. The cycle then repeats itself the following night. Because the endogenous melatonin level reaches its peak at night, this is the time period during which melatonin stimulates the most contractions. By exposing a pregnant female to a light source with sufficient intensity to suppress the endogenous melatonin level, uterine contractions are suppressed. In this context "suppressing" the endogenous melatonin level refers to either reducing the endogenous melatonin level from normal or preventing the endogenous melatonin level from rising as it normally would at night.

Clinical experiments have been performed on actual pregnant female human patients. Pregnant female human volunteers at >38 weeks of gestation were studied to determine whether exposing them to visible light would suppress their uterine contractions during the nighttime hours and whether the suppression of uterine contractions that might result from light exposure is correlated with a decrease in their endogenous melatonin levels. In the experiments, women were studied by continuously monitoring their uterine contractions from 7:00 p.m. until 7:00 a.m. under dim light. At 11:00 p.m., each woman was exposed to a 10,000 lux, full spectrum phototherapy lamp positioned about 1 meter from the woman's eyes. After about 1 hour, the lamp was turned off. The contractions were recorded by a registered obstetric nurse. The study was performed in a hospital after receiving approval from the appropriate institutional review boards.

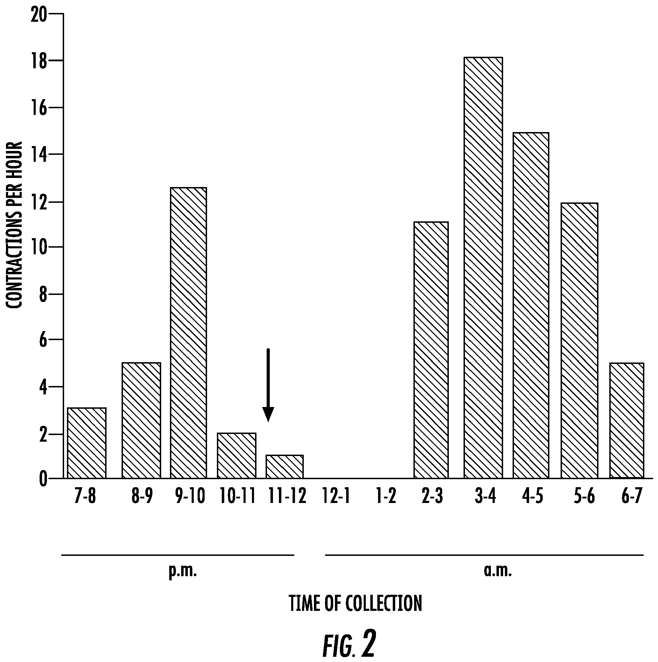

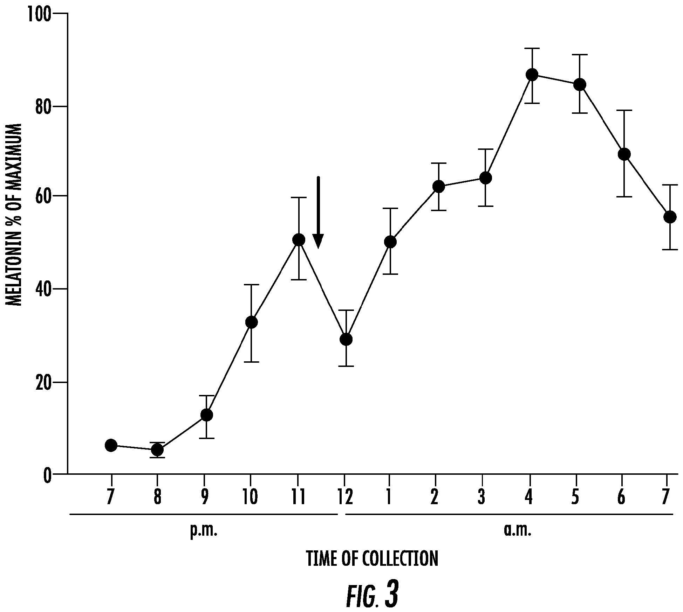

The results of these experiments are shown in FIGS. 1-3. The number of contractions two different women experienced per hour are shown in FIGS. 1 and 2. FIG. 3 shows the composite results of the melatonin levels of the women studied. The arrow in each graph represents the time at which the lamp was turned on.

The results show that, when the lamp was turned on at about 11:00 p.m., the number of contractions experienced by the women per hour decreased substantially. When the lamp was turned off after about 1 hour, the number of contractions the women experienced per hour gradually rose before eventually decreasing during the early morning hours. FIG. 3 shows that the women's endogenous melatonin levels gradually rose until 11:00 p.m. when the lamp was turned on and dropped by about 45% during the -1 hr that the lamp was activated. Once the lamp was turned off, their melatonin levels gradually rose throughout the night before decreasing during the early morning hours. When combined, the results indicate that there is indeed a direct correlation between melatonin levels and uterine contractions.

The results reveal that regular nocturnal contractions are suppressed by bright light exposure under these conditions. This finding supports the proposition that melatonin is a key zeitgeber, regulating the onset of human labor and parturition and that light can be used to regulate melatonin levels and, thereby, regulate uterine contractions.

Thus, a method and device of regulating uterine contractions involves suppressing the nocturnal endogenous melatonin level of a pregnant female experiencing uterine contractions by exposing the pregnant female during nighttime to a light source emitting visible light such as using glasses, sleep mask or other device. The intensity of the visible light is sufficient to suppress a pregnant female's endogenous melatonin level. It is also possible to regulate nocturnal uterine contractions during preterm labor by exposing a pregnant female experiencing preterm labor to a light source at night, where the light source emits visible light effective to suppress the pregnant female's endogenous melatonin level.

The light source should be of sufficient intensity and color to be able to suppress the endogenous melatonin level. In the experiment as described, the light source was a full spectrum 10,000 lux phototherapy lamp positioned about 1 meter from the pregnant female's eyes. Although this yielded good results, other light sources are suitable for use. A suitable intensity range for the light source is about 1,000 to about 10,000 lux.

The light source spectrum may be tuned to optimize the amount of melatonin suppression. One preferred light source predominantly emits blue light. Blue light in the wavelength range of about 450 to about 500 nm has been found advantageous. In one example, the pregnant female is exposed to the light during typical nocturnal or nighttime hours, preferably between about 9 p.m. to about 6 a.m. The pregnant female may be exposed to the light source continuously throughout the night or in smaller time increments during the night.

Optionally, the light source is adapted to emit light in discrete on/off cycles or pulses. The duration of the pulses and the separation between successive pulses is adjusted to obtain the desired amount of endogenous melatonin suppression.

"A Train of Blue Light Pulses Delivered Through Closed Eyelids Suppresses Melatonin and Phase Shifts the Human Circadian System," by Figueiro et al., Nature and Science of Sleep, 2013:5, 133-141, teaches that a train of blue light pulses delivered through closed eyelids suppresses melatonin and phase shifts the human circadian system. [25]

It is possible to use a personal light-emitting device for women at risk of preterm labor. Such light-emitting devices such as goggles or other glasses have already been constructed and tested with non-pregnant volunteers for sleep studies such as at Rensselaer Polytechnic Institute in Troy, New York. The light mask included goggles with machined aluminum heat sinks to dissipate heat generated by light sources. The light generated by the mask was controlled by a program that increased light levels gradually over two minutes from zero to a prescribed light level with the assumption that the temporal ramp would avoid "startling" the subjects with the light dose while they were asleep. This study describes in the open access short report by Mariana G. Figueiro and Mark S. Rea entitled, "Preliminary Evidence that Light Through the Eyelids can Suppress Melatonin and Phase Shift Dim Light Melatonin Onset," BMC Research Notes, 2012, 5:221, http://www.biomedicalcentral.com/1756-0500/5/221.

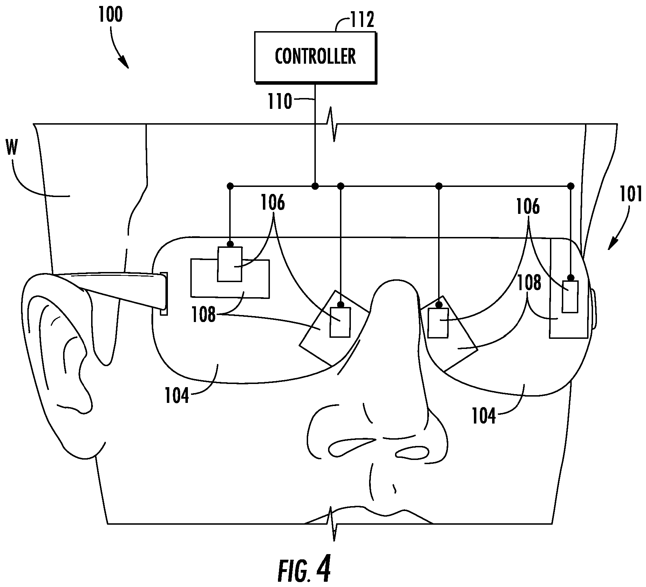

FIG. **4** shows an example of a preferred light source **100** used with a subject such as a pregnant female. The light source is formed as glasses **101** in this example, including a frame **102** supporting a pair of transparent lenses **104.** Two lights **106** are attached to each lens **104** and are oriented to shine their light towards a wearer's **W** eyes. Light diffusers **108** on the lenses **104** diffuse the light so that the lights do not appear as intense point sources of light to the wearer **W.** The diffusers **108** are made of translucent material. In this example, standard glasses may be modified.

Electrical wiring **110** in one example connects the LEDs **106** to a controller **112** that powers and controls the lights **106.** The controller **112** turns the lights **106** on and off, controls the intensity of the emitted light, and, if desired turns the lights **106** on and off in discrete on/off cycles or pulses. The controller **112** is also configured to gradually provide power to the lights **106** so that the lights **106** gradually illuminate over time. This feature is designed to prevent the wearer from waking up when the lights **106** are turned on. The lights **106** are preferably LEDs.

The controller in one example includes a source of power such as a battery. The controller can be mounted directly on the frame **102** in a position where it will not bother the wearer such as extending outward from the side of the frame between the ear and lens. In another example, the controller could be powered from a wireless power source. In yet another example, a wireless power source could be used and energy supplied directly to the lights, which could be formed as LED's that include a wireless power receiver, as an example.

The light source **100** may be worn by a pregnant female during nocturnal hours to regulate her uterine contractions, even while she sleeps. In an embodiment, the light is pulsed in discrete on/off cycles. A pulse cycle is a pulse of light for between 1-3 seconds per minute for at least about 60 minutes. Another pulse cycle is a pulse of light for about 2 seconds per minute for at least about 60 minutes. An example wavelength is between 450-500 nm, in an example, about 470 nm to about 490 nm, or about 480 nm. The light irradiance is between about 0.1 to 0.5 W/m², and in another example, between about 0.2 to 0.4 W/m², and in another example, an average of about 0.3 W/m². The light may range into the green light of about 490 or 500 to 570 nm, and thus, the light may range from 450 nm to 570 nm.

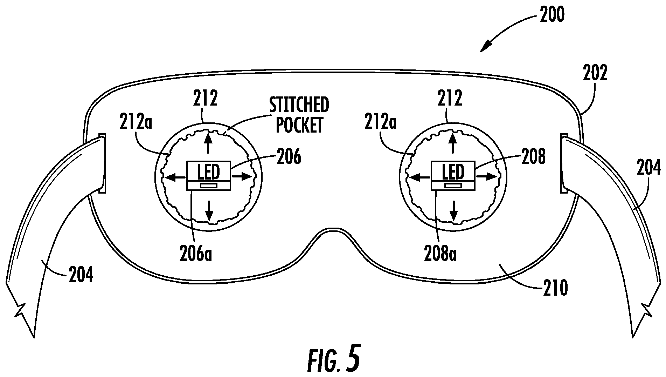

FIG. 5 is an example of a sleep mask 200 that can be used in accordance with a non-limiting example. It should be understood that the components of the sleep mask 200 could be used on glasses or goggles. The sleep mask 200 includes a flexible eye cover 202 that can be made from a variety of different flexible materials, including different fabrics and materials, but generally will be a material that is lightweight and comfortable to the user while blocking out the light. It may include an eye cavity or it can be formed without an eye cavity and, in an example, it is shaped as a more conventional sleep mask as illustrated. It can be made from different types of fabric, including polyester, cotton or other natural or man-made materials. The sleep mask 200 is formed to cover the eyes and in another example may be formed from a soft, moldable material that could also fit the contours of the face. An elastic band 204 or other type of band can be used to hold the sleep mask on the face.

The flexible eye cover 202 is configured to be worn by a pregnant female and configured to cover and shield the eyes of the wearer from ambient light. A source of light is carried by the flexible eye cover 202 and positioned to emit light onto eyelids of the wearer when asleep and penetrate the eyelids when closed. In a preferred example, the source of light is between about 450 to 500 nm and the light irradiance is between about 0.1 to 0.5 W/m². The flexible eye cover as noted before can be formed from fabric and the source of light as illustrated is formed as two light emitting diodes (LED's) **206, 208** carried by the flexible eye cover and positioned at a location where a LED can emit light onto the eyelids of the wearer when asleep.

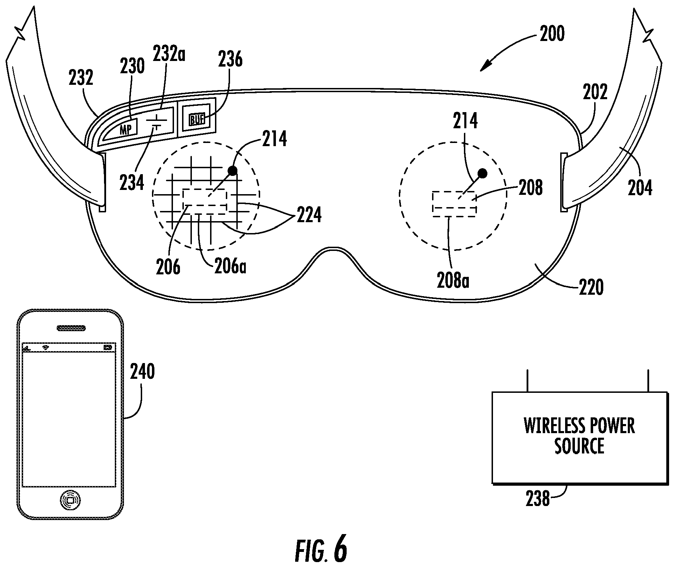

As illustrated in FIG. 5, the rear surface **210** of the flexible eye cover **202** engages the face of the wearer. A pocket **212** is formed on the rear surface at each location in an area generally where the flexible eye cover engages the eyes. The pocket **212** in this example is a circular piece of cloth stitched into the area generally where the eye cover engages the eyes, but could be formed using many different techniques. A LED **206, 208** is positioned within each pocket **212** and is moveable within the pocket to allow it to be positioned to emit light onto the eyelids of the wearer when asleep. In this example as illustrated, the pocket is shown as partially cut away **212a** to show the inside of the pocket and the LED **206, 208** positioned within the respective pocket. For example, the LED can be moved within the pocket **212** and positioned by means of an adjuster member **214** as shown in the front view of FIG. 6 and shows a short rod member as the adjuster member extending outward from the front face **220** of the flexible eye cover at the pocket and configured to be grabbed by the wearer to move or manipulate manually a respective LED within the respective pocket into a position adjacent an eye to ensure that the light from the LED is emitted onto the closed eyelid of the wearer when asleep.

The front surface of the cloth or other material forming the pocket **212** may include guide tracks such as fine cuts **224** to allow the adjuster member **214** to be moved vertically or horizontally and position the LED in a desired location that is amenable for use by the user or pregnant female to allow the best position where light shines onto the eyelid. The pocket **212** can be positioned on the front or rear surface of the flexible eye cover and in the illustrated example is shown on the rear surface, but can be on the front surface. The pocket cloth can be made from a different material than that used for the flexible eye cover and can be made more clear to allow the LED light to pass through the material more readily.

A processor **230** or other controller is connected to each LED **206, 208** by wired or wireless connection and configured to activate the LED **206, 208** in a manner to decrease endogenous melatonin levels and aid in regulating uterine contractions in the pregnant female. The processor **230** may be carried in another pocket **232** formed in this example as shown in FIG. 6 in a similar manner as pockets **212.** It is shown in a partial cut away **232a** and may include a battery **234** or a wireless power inductor or receiver **236** connected to the processor **230** that receives wireless power through a separate wireless power source **238.** The LED's **206, 208** may include a small wireless power module **206a, 208a** that receives wireless power such that the wireless power itself drives the LED in a predetermined manner with a ramp up of power and intensity and pulsing. A wired connection may be used, of course. Programming of the processor may be through another controller such as a wireless device **240** as illustrated such as a cellular phone, or be preprogrammed when the sleep mask is purchased to be used by the user or pre-programmed by a medical specialist especially for the user as a pregnant female.

FIG. 6 shows an example of the wireless device **240** that can be used to program the processor with a special application program that could be downloaded on an iphone as an example. A wireless power source **238** is illustrated as shown in FIG. 6. In an example, the processor is programmed so that the illumination is increased over a time period of between about 5 to 30 seconds. The light may be pulsed in discrete on and off cycles such as between 1 to 3 seconds per minute for about 30 to about 60 minutes as noted before.

The processor **230** as a controller may emit pulses at certain times of the night for a certain length of time. The processor **230** can be user programmable via the separate wireless device **240** or preprogrammed when purchased by a user. A mobile device such as a cellular phone, iphone or other wireless devices is used as a controller in one example to transmit information to the processor for alternate timings and light intensity profiles. Any battery **234** as used would be small for LED actuation and could be rechargeable through a recharge port in an example. White light would be difficult since it may not be feasible especially with a partner sleeping near the user and it could be bothersome to the user. The blue light is a better light and just as effective as white light and has been found effective to suppress melatonin. Also, white light may not penetrate the eyelids as well as the blue light does. The cycling can range from zero power up to full power in ten seconds as an example and can remain on for a minute or half a minute and then go off again through the night. It is possible to operate the mask with a phase response curve to shift the clock eastward or westward by a predetermined number of hours depending on when the light is given. If the light is given in the early evening hours, then time is truncated opposite from light given in the early morning to truncate for the natural low point. It is possible to accelerate the clock. In one example, light pulses are given in the evening and morning for one-half hour to one hour each time and with intermittent ramping and intermittent operation to reduce the truncation. These times can vary.

There are different types of wireless power systems that could be implemented in accordance with a non-limiting example. It is possible to use inductive coupling that uses magnetic fields that are a natural part of the current movement through a wire. For example, when the electrical current moves through a wire, it creates a circular magnetic field around the wire and bends the wire into a coil that amplifies the magnetic field. The more loops the coil has, the larger the field will be produced. When a second coil of wire is placed in the magnetic field, the field can include a current in the wire. It is also possible to use resonance and wireless power conduction can take place differently when the electromagnetic fields around the coils resonate at the same frequency. The inductor can be formed as a curved coil of wire and a capacitance plate can hold a charge and attach to each end of the coil. When electricity travels through the coil, it resonates and the resonant frequency is a product of the inductance of the coil and capacitance of the plates. The electricity can tunnel from one coil to the other as it travels along the electromagnetic wave if both have the same resonant frequency. Electromagnetic induction is proportional to the intensity of the current and voltage in the conductor, which produces the fields and to the frequency. Other wireless power techniques may be used.

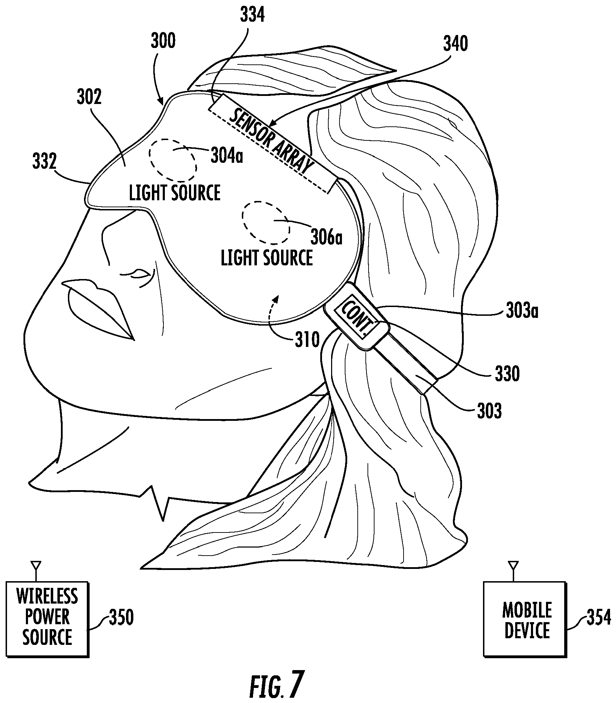

Referring now to FIG. 7, there is shown another embodiment of the sleep mask **300,** in accordance with a non-limiting example. The sleep mask **300** is formed of flexible material to form a flexible eye cover **302** as in the embodiment of FIGS. 5 and 6 and limits ambient light into the eyes of the wearer. It includes an elastic band **303** and rear surface **310** that engages the face and forehead of the wearer. The elastic band **303** is mounted to a support **303a.** This embodiment of the sleep mask **300** may include a pocket, LED adjustor, and other components similar to the sleep mask shown in FIGS. 5 and 6. The sleep mask **300** is configured to be worn by the user, in this example a late-term pregnant female. A light source is carried by the sleep mask **300,** and in this example, light sources **304a, 306a** are carried by the sleep mask **300** to direct light towards each of the eyes as illustrated. The light sources **304a, 306a** are configured to direct light preferably through the eyelids of the late-term pregnant human female while sleeping to reduce uterine contractions occurring at night in the late-term pregnant human female and suppress the regular nocturnal endogenous melatonin secretions of the late-term pregnant human female experiencing uterine contractions. The light sources **304a, 306a** in an example are configured to direct into the eyes of the pregnant human female during the night light having a wavelength of about 450 to 570 nm and an irradiance of about 0.1 to 0.5 W/m². In another example, it may be an intensity of about 1,000 to 10,000 lux. This wavelength range encompasses the preferred blue and into the green color. The sleep mask functions may be controlled by a controller **330** mounted on the outer portion of the elastic band **303** or other location. The sleep mask **300** includes a periphery **334** and a top peripheral portion **332** that engages the forehead.

In this example, a sensor array **340** is carried along the top peripheral portion **334** and positioned to engage the forehead of the wearer, which typically may be a late-term pregnant female. Of course, the use of the sleep mask **300** is not limited to later-term pregnant females and other wearers may use the sleep mask **300** and take advantage of the sensor array **340** and other functions, such as use of emitted light to aid in shifting the biological time clock of the wearer. Thus, the sleep mask **300** may be used by others in long distance air flights or other situations where jet lag could be problematic as explained below. A portable wireless device **350** is illustrated, for example a cell phone, and wireless power device **354** that may interoperate with the sleep mask **300** as will be explained below.

In this example and as explained in greater detail below, the sensor array **340** may include individual sensors for determining different body functions, including EEG, EMG, EOG, temperature, body movement or use as an oximeter for blood oxygen sensing. Other sensors may be included. These sensors interoperate with the controller **330** and may be wired or wireless.

It has been found that green light may also be effective to decrease endogenous melatonin levels and in regulating or reducing uterine contractions. Green light has a wavelength of about 495 or 500 to 570 nm and a frequency of about 575 to 525 THz. Green light is between the blue and yellow in the spectrum of visible light. Although the green light may not be as effective as the blue light, it still may be used. As noted before, the light sources **304a, 306a** for each eye may be timed for exposure between 9:00 p.m. and 6:00 a.m. and may be configured to direct light intermittently into the eyes of the pregnant human female during the night. The blue light of 450 to 500 nm or 450 to 490 nm is preferred to be directed into the eyes of the pregnant human female. The controller **330** is carried by the sleep mask **300,** and in the example shown in FIG. 7, carried at the support **303a** of the elastic band **303,** but can be carried at other areas of the sleep mask **300.**

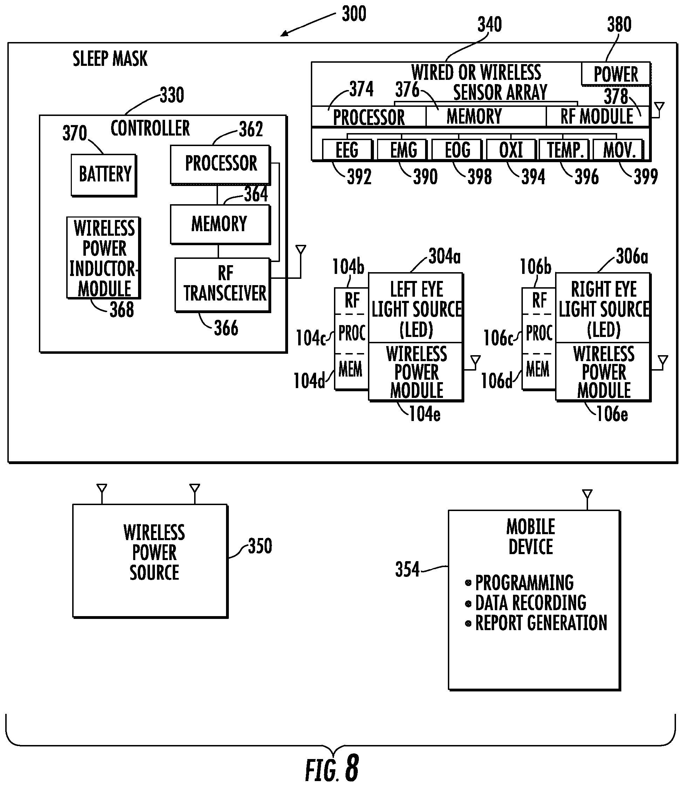

As shown in FIG. 8, the controller **330** includes a processor **360** and memory **362.** The controller **330** is connected to each of the light sources **304a, 306a** by either wired or wireless connection and configured to control the time, wavelength and irradiance of light emitted from the light sources. It may control the intensity. An example light source is a LED or multiple LED's for each light source that can be controlled in terms of its on/off time such as intermittent on/off operation and have its wavelength and intensity changed to allow a range of wavelengths such as visible light, blue light, green light, or any combination.

The controller **330** includes the processor **362** and a memory **364** coupled thereto and configured to store instructions regarding the operation of the sensor array **340,** for example, or configure the light sources **304a, 306a** so that the time, wavelength and irradiance of light emitted from the light sources can be controlled. It may control intensity.

As illustrated, the processor **362** and memory **364** interoperate with a RF transceiver **366** that may send and receive different instructions and data. For example, the RF transceiver **366** may send instructions to one of the light sources **304a, 306a** to change its operation as to intermittent flashing, duration, irradiance, intensity and/or wavelength. This can be accomplished in one example using an RF module **304b, 306b,** processor **304c, 306c,** and memory **304d, 306d** having stored instructions and configuring each light source 304a, 306a. Each light source 304a, 306a may include a wireless power module 304e, 306e and be operable with a wireless power inductor module 368 at the controller 330 and receive inductive power from the wireless power source 350. A battery 370 could be used at the controller 330 instead. Batteries could be used at the sleep mask for the LED's and other components. The sensor array 340 may also include a processor 374, memory 376, and RF module 378 and interoperate with the controller 330. It may also include wireless power module 380 or a battery. It should be understood that each light source 304a, 306a may be formed from one or more light emitting devices, such as light emitting diodes, which may be controlled so that not only wavelength, but the intensity of emitted light may be controlled, if multiple light emitting devices are used, the combination and range of wavelength and intensity could be varied for specific persons. For example, each light source 304a, 306a could be formed from an array of LED's, each having a range of wavelengths and variable intensity. Records and data could be recorded and analyzed since data is obtained from the sensor array 340.

The controller 330 may be configured to direct light intermittently over a period of time at night into the eyes and at a wavelength sufficient to shift the biologic time clock of the human female. For example, the light sources 304a, 306a may be configured to have a wavelength in the range of 450 to 570 nm corresponding to visible light. The different intermittent operation or phases of light can be timed to phase-shift the biological time clock of the wearer. In another example, the tight irradiance is between about 0.1 to 0.5 W/m² for blue/green or blue light. It is possible to use a wireless power inductor module 368 or battery 370. As a result, the processor 362 and RF transceiver **366,** the light sources **304a, 306a** and other components may receive wireless power transmitted from the wireless power source **350** that is external in this example or even part of the controller. The battery **370** may be used to power the induction module **368.** As an alternative, the sensor array **340,** the light sources **304a, 306a,** or other components may each include batteries. The illumination of light may be increased over a predetermined period of time and increased over a time period of between about 5 to 30 seconds. The light may be pulsed in discrete on and off cycles, and in an example, is pulsed for about 1-3 seconds per minute for about 30 to about 60 minutes. In an example, the light source is formed as a light emitting diode or series of light emitting diodes at each light source.

It is possible to program the controller **330** from the wireless mobile device **354** and communicate for programming, data recording and record keeping as illustrated in FIG. 8.

The mobile wireless device **354** may be used to program the processor **362** in the controller **330** with a special application program that could be downloaded on the device as an example. In an example, the processor **362** is programmed so that the illumination of the light sources **304a, 306a** is increased over a time period of between about 5 to 30 seconds. The light may be pulsed in discrete on and off cycles such as between 1 to 3 seconds per minute for about 30 to about 60 minutes as noted before.

The processor **362** as part of the controller **330** may direct the light sources **304a, 306a** to emit pulses at certain times of the night for a predetermined length of time. The processor **362** can be user programmable via the separate wireless device **354** or preprogrammed when purchased by a user. A mobile wireless device **354** such as a cellular phone, iPhone or other wireless devices may be used as a controller **330** in one example to transmit information to the processor **362** via the RF transceiver **366** or even directly to the sensor array **340** via its RF module **378** or directly to the light sources **304a, 306a,** for alternate timing and light intensity or irradiance profiles and sensor configurations. Any battery, if used, would be small for LED or other light source actuation, and could be rechargeable through a recharge port in an example. White light may not be feasible especially with a partner sleeping near the user and it could be bothersome to the user. The blue light is the better light and just as effective or more effective as white light and has been found effective to suppress melatonin and overtones with green light may be used. Also, white light may not penetrate the eyelids as well as the blue light does. The cycling can range from zero power up to full power in ten seconds as an example and can remain on for a minute or half a minute and then go off again through the night. It is possible to operate the sleep mask **300** with a phase response curve to shift the clock eastward or westward by a predetermined number of hours depending on when the light is given. If the light is given in the early evening hours, then time is truncated opposite from light given in the early morning to truncate for the natural low point. It is possible to accelerate the clock. In one example, light pulses are given in the evening and morning for one-half hour to one hour each time and with intermittent ramping and intermittent operation to reduce the truncation. These times can vary.

It is possible that the controller **330** can control the light emissions to allow an artificial dawn or wake-up by light as by being timed to certain portions of the night or for napping as noted before. The controller **330** may control timed stages of sleep for short or long power naps or even into a deep sleep for better body and mind renewal over a day or night.

The sensor array **340** has various sensors as noted before. An example is an EMG sensor **390** to measure EMG (electromyogram) for muscle activity, including different twitches and movements of the face and even teeth grinding that occur during sleep. This can aid in determining if there is proper REM sleep. An EEG sensor **392** could measure the EEG (electroencephalography) for brain waves and determine different sleep stages. It is possible to include an oximetry sensor **394** to determine a pulse rate and blood oxygen levels. Different sensors could be used, including two sensors or electrodes that emit two different wavelengths such as red and near-infrared and measure the change in light absorbance at each wavelength. For example, more infrared light is absorbed by oxygenated hemoglobin, which allows the red light to pass through. Any deoxygenated hemoglobin will absorb more red light, but allow the infrared light to pass through. The blood oxygen levels and heart rate may thus be determined.

It is possible to measure body temperature during the night using a temperature sensor **396** and determine melatonin concentration. It is possible to use single-channel EEG electrodes at the EEG sensor **392.** It is possible to use dry electrodes and other electrodes as an EOG sensor **398** to measure EOG (electrooculogram) to record eye movement and help determine if a REM sleep state has been established. Other movement sensors **399** may be used. The EMG signals may be emitted at a higher frequency than any EEG signals to determine the differences and prevent signal interference. The controller **330** may use different processing techniques to distinguish among EMG-artifacts and EEG signals. Pulse oximetry measurements can be used to diagnose for sleep apnea. Different oximeter sensors may be used, including reflectance-based sensors. Dry electrodes may be used that incorporate micro-needles to penetrate layers of the skin. MEMS technology may be used in conjunction with the electrodes. Different processing techniques, including semiconductor processing techniques, may be used to form different electrodes, including dry electrodes.

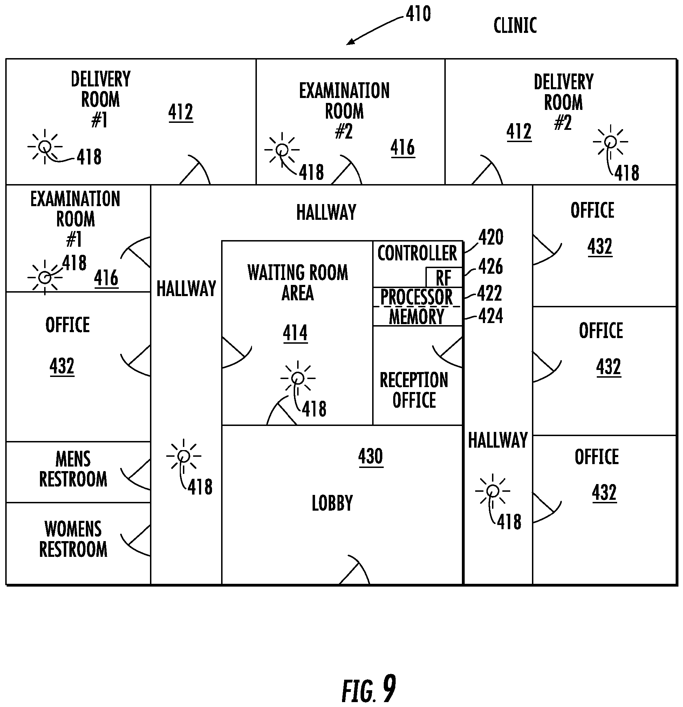

FIG. 9 is a fragmentary plan view of a facility **410** as a pregnancy clinic forming a system, in accordance with a non-limiting example, that may reduce uterine contractions occurring in a late-term pregnant human female when that female visits the facility. For example, different areas of the facility such as the delivery room **412,** waiting room area **414,** or examination room **416** may each contain a light source **418.** A controller **420** may be connected to the light source **418** and configured to control the light source to direct ambient light into the eyes of the late-term pregnant human female for reducing uterine contractions in the late-term pregnant human female and suppressing the regular endogenous melatonin secretions in the late-term pregnant human female experiencing uterine contractions. When the patient walks into these rooms, the patient is exposed to the blue or blue/green ambient light. The controller **420** is shown incorporating a processor **422,** memory **424,** and RF module **426** in one of the offices and is configured to control the light source **418** to direct into the eyes of the pregnant human female the ambient light having a wavelength of about 450 to 570 nm, and in an example, having an intensity of about 1,000 to 10,000 lux or light irradiance of about 0.1 to 0.5 W/m². Wireless RF or wired connections from the controller 420 to light sources 418 may be used. Although the wider range of ambient light is disclosed, it is possible to use the blue ambient light having a wavelength of about 450 to 500 nm or 450 to 490 nm. In the illustrated example shown in FIG. 6, the delivery room 412, waiting room 414, and the three examination rooms 416 each would have a light source 4 8 to direct the ambient light into the eyes of the pregnant human female and preferably at a wavelength of about 450 to 570 nm, and in an example, the blue wavelength of about 450 to 500 nm.

It is possible that the lobby 430 and other offices 432 could include a light source that emits the ambient light to operate and reduce uterine contractions, !n certain instances, it may be possible to use a light source in certain areas that is modified so no blue light or green light, i.e., the range of frequencies of about 450 to 570 nm, are not emitted so that uterine contractions are not reduced and pregnancy and contractions occur and delivery of a body occurs without delay.

The invention has been described above with reference to preferred embodiments. Unless otherwise defined, all technical and scientific terms used herein are intended to have the same meaning as commonly understood in the art to which this invention pertains and at the time of its filing. Although various methods and materials similar to those described herein can be used in the practice or testing of the present invention, suitable methods and materials are described. However, the skilled should understand that the methods and materials used and described are examples and may not be the only ones suitable for use in or with the invention. Various modifications of the embodiments described here can be made without departing from the scope of the invention as described above and as defined in the appended claims.

### REFERENCES

1. Plaut SM, Grota LJ, Ader R, Graham CW. Effects of handling and the light-dark cycle on time of parturition in the rat. Lab Anim Care. 1970; 20(3):447-453.
2. Boer K, Lincoln DW, Swaab DF. Effects of electrical stimulation of the neurohypophysis on labour in the rat. J Endocrinol. 1975; 65(2):163-176.
3. Lincoln DW, Porter DG. Timing of the photoperiod and the hour of birth in rats. Nature. 1976; 260(5554):780-781.
4. Siegel HI, Greenwald GS. Prepartum onset of maternal behavior in hamsters and the effects of estrogen and progesterone. Horm Behav. 1975; 6(3):237-245.
5. Glattre E, Bjerkedal T. The 24-hour rhythmicity of birth: a population study. Acta Obstet Gynecol Scand. 1983; 62:31-36.
6. Cooperstock M, England JE, Wolfe RA. Circadian incidence of labor onset hour in preterm birth and chorioamnionitis. Obstet Gynecol. 1987; 70(6):852-855.
7. Cagnacci A, Soldani R, Melis GB, Volpe A. Diurnal rhythms of labor and delivery in women: modulation by parity and seasons. Am J Obstet Gynecol. 1998; 178(1 pt 1):140-145.
8. Lindow SW, Jha RR, Thompson JW. 24-hour rhythm to the onset of preterm labour. Br J Obstet Gynecol. 2000; 107(9):1145-1148.
9. Vatish M, Steer PJ, Blanks AM, Hon M, Thornton S. Diurnal variation is lost in preterm deliveries before 28 weeks of gestation. Br J Obstet Gynecol. 2010; 117(6):765-767.
10. lams JD, Newman RB, Thom EA, et al. Frequency of uterine contractions and the risk of spontaneous preterm delivery. N Engl J Med. 2002; 346(4):250-255.
11. Harbert GM Jr. Biorhythms of the pregnant uterus (Macaca mulatta). Am J Obstet Gynecol. 1977; 129(4):401-408.
12. Morgan MA, Silavin SL, Wentworth RA, et al. Different patterns of myometrial activity and 24-h rhythms in myometrial contractility in the gravid baboon during the second half of pregnancy. Biol Reprod. 1992; 46(6):1158-1164.
13. Honnebier MBOM, Myers T, Figueroa JP, Nathanielsz PW. Variations inmyometrial response to intravenous oxytocin administration at different times of the day in the pregnant rhesus monkey. Endocrinology. 1989; 125(3):1498-1503.
14. Ducsay CA, Yellon SM. Photoperiod regulation of uterine activity and melatonin rhythms in the pregnant rhesus macaque. Biol Reprod. 1991; 44(6):967-974.
15. Main DM, Grisso JA, Wold T, Snyder ES, Holmes J, Chiu G. Extended longitudinal study of uterine activity among low-risk women. Am J Obstet Gynecol. 1991; 165(5 pt 1):1317-1322.
16. Zahn V, Hattensperger W. Circadian rhythm of pregnancy contractions. Z Geburtshilfe Perinatol. 1993; 197(1):1-10.
17. Farber DM, Giussani DA, Jenkins SL, et al. Timing of the switch from myometrial contractures to contractions in late-gestation pregnant rhesus monkeys as recorded by myometrial electromyogram during spontaneous term and androstenedione-induced labor. Biol Reprod. 1997; 56(2):557-562.
18. Prasai MJ, Pernicova I, Grant PJ, Scott EM. An endocrinologist's guide to the clock. J Clin Endocrinol Metab. 2011; 96(4):913-922.
19. Messager S, Hazlerigg DG, Mercer JG, Morgan PJ. Photoperiod differentially regulates the expression of Per1 and ICER in the pars tuberalis and the suprachiasmatic nucleus of the Siberian hamster. Eur J Neurosci. 2000; 12(8):2865-2870.
20. von Gall C, Garabette ML, Kell CA, et al. Rhythmic gene expression in pituitary depends on heterologous sensitization by the neurohormone melatonin. Nat Neurosci. 2002; 5(3):234-238.
21. Imbesi M, Dirim DA, Yildiz S, et al. The melatonin receptor MT1 is required for the differential regulatory actions of melatonin on neuronal clock gene expression in striatal neurons in vitro. J Pineal Res. 2009; 46(1):87-94.
22. Balsalobre A. Clock genes in mammalian peripheral tissues. Cell Tissue Res. 2002; 309(1):193-199.
23. Dickmeis T. Glucocorticoids and the circadian clock. J Endocrinol. 2009; 200(1):3-22.
24. Kiessling S, Eichele G, Oster H. Adrenal glucocorticoids have a key role in circadian resynchronization in a mouse model of jet lag. J Clin Invest. 2010; 120(7):2600-2609.
25. Figueiro, M.G., Bierman, A., and Rea, M.S. A train of blue light pulses delivered through closed eyelids suppresses melatonin and phase shifts the human circadian system. Nature and Science of Sleep 2013:5 133-41.

## Claims

1. A sleep mask (200,300) for decreasing endogenous melatonin levels to aid in suppressing uterine contractions in a pregnant female wearer, comprising:
a flexible eye cover (202,302) configured to be worn by a pregnant female wearer and configured to cover and shield the eyes of the wearer from ambient light;
at least one light source (100,304a,306a) carried by the flexible eye cover (202,302) and positioned to emit light having a wavelength of 450 to 570 nm onto the eyelids of the wearer when asleep and penetrate the eyelids when closed; and
a controller (112,330) connected to the at least one light source (100,304a,306a) and configured to activate the at least one light source (100,304a,306a):
**characterised in that** the light irradiance emitted from the at least one light source (100,304a,306a) is between about 0.1 to 0.5 W/m².

2. A sleep mask (200,300) according to Claim 1, further comprising at least one sensor (340) carried by the flexible eye cover (202,302) and positioned to engage the skin of the wearer and measure at least one of EMG (electromyogram) (390), EEG (electroencephalography) (392), and temperature (396): wherein the controller (112,330) is connected to the at least one sensor (340) and configured to control at least one of the duration, wavelength and irradiance of the light emitted from the light source (100,304a,306a) based on at least one of sensed EMG, EEG and temperature.

3. A sleep mask (200,300) according to Claim 2, wherein the at least one sensor (340) comprises a sensor array (340) comprising the EEG sensor (392), the EMG sensor (390) and the temperature sensor (396).

4. A sleep mask (200,300) according to Claim 3, wherein the at least one sensor (340) further comprises at least one of an EOG (electrooculogram) sensor (398) and a pulse oximetry sensor (394).

5. A sleep mask (200,300) according to any of the preceding claims, wherein the wavelength of the light emitted from the at least one light source (100,304a,306a) is between about 450 to 500 nm.

6. A sleep mask (200,300) according to any of the preceding claims, wherein the controller (112,330) is configured to increase the illumination of the light emitted from the at least one light source (100,304a,306a) over a predetermined period of time.

7. A sleep mask (200,300) according to Claim 6, wherein the illumination is increased over the period of time between about 5 to 30 seconds.

8. A sleep mask (200,300) according to any of the preceding claims, wherein the controller (112,330) is configured to pulse the light emitted from the at least one light source (100,304a,306a) in discrete on and off cycles.

9. A sleep mask (200,300) according to Claim 8, wherein the light is pulsed for about 1 to 3 seconds per minute for about 30 to about 60 minutes.

10. A sleep mask (200,300) according to any of the preceding claims, wherein the light source (100,304a,306a) comprises a light emitting diode (LED) (206,208) carried by the flexible eye cover (202,302) and positioned at a location where an LED can emit light onto the eyelids of the wearer when asleep.

11. A sleep mask (200,300) according to any of the preceding claims, wherein the controller (112,330) is configured to control the at least one light source (100,304a,306a) to direct light intermittently over a period of time into the eyes and at a wavelength to shift the biologic time clock of the human female.

## Patentansprüche

1. Schlafbrille (200, 300) zum Senken endogener Melatoninspiegel, um bei der Unterdrückung von Gebärmutterkontraktionen bei einer schwangeren Trägerin zu unterstützen, umfassend:
eine flexible Augenabdeckung (202, 302), die dazu konfiguriert ist, durch eine schwangere Trägerin getragen zu werden, und dazu konfiguriert ist, die Augen der Trägerin abzudecken und vor Umgebungslicht abzuschirmen;
mindestens eine Lichtquelle (100, 304a, 306a), die durch die flexible Augenabdeckung (202, 302) aufgenommen ist und so positioniert ist, dass sie Licht, das eine Wellenlänge von 450 bis 570 nm aufweist, auf die Augenlider der Trägerin emittiert, wenn diese schläft, und die Augenlider durchdringt, wenn sie geschlossen sind; und
eine Steuerung (112, 330), die mit der mindestens einen Lichtquelle (100, 304a, 306a) verbunden ist und dazu konfiguriert ist, die mindestens eine Lichtquelle (100, 304a, 306a) zu aktivieren:
**dadurch gekennzeichnet, dass** die von der mindestens einen Lichtquelle (100, 304a, 306a) emittierte Lichtstrahlungsintensität zwischen etwa 0,1 und 0,5 W/m² liegt.

2. Schlafbrille (200, 300) nach Anspruch 1, ferner mindestens einen Sensor (340) umfassend, der durch die flexible Augenabdeckung (202, 302) aufgenommen ist und so positioniert ist, dass er mit der Haut der Trägerin in Eingriff kommt und mindestens eines von EMG (Elektromyogramm) (390), EEG (Elektroenzephalographie) (392) und Temperatur (396) misst: wobei die Steuerung (112, 330) mit dem mindestens einen Sensor (340) verbunden ist und dazu konfiguriert ist, mindestens eines von der Dauer, Wellenlänge und Strahlungsintensität des von der Lichtquelle (100, 304a, 306a) emittierten Lichts basierend auf mindestens einem von dem erfassten EMG, der erfassten EEG und Temperatur zu steuern.

3. Schlafbrille (200, 300) nach Anspruch 2, wobei der mindestens eine Sensor (340) ein Sensorarray (340) umfasst, das den EEG-Sensor (392), den EMG-Sensor (390) und den Temperatursensor (396) umfasst.

4. Schlafbrille (200, 300) nach Anspruch 3, wobei der mindestens eine Sensor (340) ferner mindestens eines von einem EOG(Elektrookulogramm)-Sensor (398) und einem Pulsoximetriesensor (394) umfasst.

5. Schlafbrille (200, 300) nach einem der vorhergehenden Ansprüche, wobei die Wellenlänge des von der mindestens einen Lichtquelle (100, 304a, 306a) emittierten Lichts zwischen etwa 450 und 500 nm liegt.

6. Schlafbrille (200, 300) nach einem der vorhergehenden Ansprüche, wobei die Steuerung (112, 330) dazu konfiguriert ist, die Beleuchtungsstärke des von der mindestens einen Lichtquelle (100, 304a, 306a) emittierten Lichts über einen vorbestimmten Zeitraum zu erhöhen.

7. Schlafbrille (200, 300) nach Anspruch 6, wobei die Beleuchtungsstärke über den Zeitraum zwischen etwa 5 und 30 Sekunden erhöht wird.

8. Schlafbrille (200, 300) nach einem der vorhergehenden Ansprüche, wobei die Steuerung (112, 330) dazu konfiguriert ist, das von der mindestens einen Lichtquelle (100, 304a, 306a) emittierte Licht in getrennten Ein- und Aus-Zyklen impulsweise auszustrahlen.

9. Schlafbrille (200, 300) nach Anspruch 8, wobei das Licht etwa 30 bis etwa 60 Minuten lang etwa 1 bis 3 Sekunden pro Minute impulsweise ausgestrahlt wird.

10. Schlafbrille (200, 300) nach einem der vorhergehenden Ansprüche, wobei die Lichtquelle (100, 304a, 306a) eine lichtemittierende Diode (LED) (206, 208) umfasst, die durch die flexible Augenabdeckung (202, 302) aufgenommen ist und an einer Stelle positioniert ist, an der eine LED Licht auf die Augenlider der Trägerin, wenn diese schläft, emittieren kann.

11. Schlafbrille (200, 300) nach einem der vorhergehenden Ansprüche, wobei die Steuerung (112, 330) dazu konfiguriert ist, die mindestens eine Lichtquelle (100, 304a, 306a) so zu steuern, dass sie Licht über einen Zeitraum hinweg intermittierend und mit einer Wellenlänge in die Augen leitet, um die biologische Uhr der Frau zu verschieben.

## Revendications

1. Masque de sommeil (200, 300) destiné à diminuer les taux de mélatonine endogène de manière à aider à supprimer les contractions utérines chez une femme enceinte utilisatrice, comprenant :
un couvre-œil souple (202, 302) conçu pour être porté par une femme enceinte utilisatrice et conçu pour couvrir et protéger les yeux de l'utilisatrice de la lumière ambiante ;
au moins une source de lumière (100, 304a, 306a) portée par le couvre-œil souple (202, 302) et positionnée de manière à émettre une lumière comportant une longueur d'onde de 450 à 570 nm sur les paupières de l'utilisatrice lorsqu'elle dort et pénétrant les paupières lorsqu'elles sont fermées ; et
un dispositif de commande (112, 330) connecté à ladite au moins une source de lumière (100, 304a, 306a) et conçu pour activer ladite au moins une source de lumière (100, 304a, 306a) :
**caractérisé en ce que** l'irradiance lumineuse émise par ladite au moins une source de lumière (100, 304a, 306a) est comprise entre environ 0,1 et 0,5 W/m².

2. Masque de sommeil (200, 300) selon la revendication 1, comprenant en outre au moins un capteur (340) porté par le couvre-œil souple (202, 302) et positionné de manière à venir en contact avec la peau de l'utilisatrice et mesurer au moins un élément parmi l'EMG (électromyogramme) (390), l'EEG (électro-encéphalogramme) (392) et la température (396) : ledit dispositif de commande (112, 330) étant connecté audit au moins un capteur (340) et conçu pour contrôler au moins une paramètre parmi la durée, la longueur d'onde et l'irradiance de la lumière émise par la source de lumière (100, 304a, 306a) sur la base d'au moins un élément parmi l'EMG, l'EEG et la température détectés.

3. Masque de sommeil (200, 300) selon la revendication 2, ledit au moins un capteur (340) comprenant un réseau de capteurs (340) comprenant le capteur d'EEG (392), le capteur d'EMG (390) et le capteur de température (396).

4. Masque de sommeil (200, 300) selon la revendication 3, ledit au moins un capteur (340) comprenant en outre au moins un capteur parmi un capteur d'EOG (électro-oculogramme) (398) et un capteur d'oxymétrie de pouls (394).

5. Masque de sommeil (200, 300) selon l'une quelconque des revendications précédentes, ladite longueur d'onde de la lumière émise par ladite au moins une source de lumière (100, 304a, 306a) étant comprise entre environ 450 et 500 nm.

6. Masque de sommeil (200, 300) selon l'une quelconque des revendications précédentes, ledit dispositif de commande (112, 330) étant configuré pour augmenter l'éclairage de la lumière émise par ladite au moins une source de lumière (100, 304a, 306a) sur une période de temps prédéfinie.

7. Masque de sommeil (200, 300) selon la revendication 6, ledit éclairage étant augmenté sur une période de temps comprise entre environ 5 et 30 secondes.

8. Masque de sommeil (200, 300) selon l'une quelconque des revendications précédentes, ledit dispositif de commande (112, 330) étant configuré pour pulser la lumière émise par ladite au moins une source de lumière (100, 304a, 306a) dans des cycles d'activation et de désactivation discrets.

9. Masque de sommeil (200, 300) selon la revendication 8, ladite lumière étant pulsée pendant environ 1 à 3 secondes par minute pendant environ 30 à environ 60 minutes.

10. Masque de sommeil (200, 300) selon l'une quelconque des revendications précédentes, ladite source de lumière (100, 304a, 306a) comprenant une diode électroluminescente (DEL) (206, 208) portée par le couvre-œil souple (202, 302) et positionnée à un emplacement où une LED peut émettre de la lumière sur les paupières de l'utilisatrice lorsqu'elle dort.

11. Masque de sommeil (200, 300) selon l'une quelconque des revendications précédentes, ledit dispositif de commande (112, 330) étant conçu pour commander ladite au moins une source de lumière (100, 304a, 306a) de manière à diriger la lumière par intermittence sur une période de temps dans les yeux et à une longueur d'onde permettant de décaler l'horloge biologique de la femme.
